(19) Europäisches Patentamt
European Patent Office
Office européen des brevets

(11) EP 0 895 995 B1

# (12) FASCICULE DE BREVET EUROPEEN

(45) Date de publication et mention de la délivrance du brevet:
**05.06.2002 Bulletin 2002/23**

(51) Int Cl.[7]: **C07D 519/00**, A61K 31/44 // (C07D519/00, 471:00, 471:00)

(21) Numéro de dépôt: **98401979.4**

(22) Date de dépôt: **04.08.1998**

(54) **Nouveaux composés de bis [4,3-b]carbazole leur procédé de préparation et les compositions pharmaceutiques qui les contiennent**

Bis Pyrido[4,3-B]Carbazolverbindungen, Verfahren zu ihrer Herstellung und diese enthaltende pharmazeutische Zubereitungen

Bis pyrido[4,3-b]carbazole compounds, process for their preparation and pharmaceutical compositions containing them

(84) Etats contractants désignés:
**AT BE CH DE DK ES FI FR GB GR IE IT LI LU NL PT SE**

(30) Priorité: **06.08.1997 FR 9710066**

(43) Date de publication de la demande:
**10.02.1999 Bulletin 1999/06**

(73) Titulaire: **LES LABORATOIRES SERVIER**
**92200 Neuilly sur Seine (FR)**

(72) Inventeurs:
- **Guillonneau, Claude**
  **92415 Clamart (FR)**
- **Charton, Yves**
  **92330 Sceaux (FR)**
- **Atassi, Ghanem**
  **92210 Saint Cloud (FR)**
- **Pierre, Alain**
  **78580 Les Alluets Le Roi (FR)**
- **Guilbaud, Nicolas**
  **78170 La Celle Saint Cloud (FR)**

(56) Documents cités:
**EP-A- 0 591 058**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne de nouveaux composés de bis pyrido [4,3-b] carbazole, leur procédé de préparation et les compositions pharmaceutiques qui les contiennent.

Les composés de la présente invention trouvent leur utilisation en thérapeutique grâce à leur activité antitumorale.

Certains dérivés de la famille de l'olivacine ou de l'ellipticine sont déjà connus pour leurs propriétés anticancéreuses, cf la demande de brevet EP O 591 058 A1.

Les besoins de la thérapeutique exigent le développement constant de nouveaux agents anticancéreux dans le but d'obtenir des molécules à la fois plus actives et plus spécifiques.

La présente invention concerne des dérivés de l'olivacine ou de l'ellipticine, présentant par rapport aux composés les plus proches de l'art antérieur, une originalité structurale (puisque deux groupes, olivacine ou ellipticine identiques sont reliés par leur position 9 grâce à un enchaînement alkyl dicarboxy), qui s'accompagne d'une excellente activité antitumorale notamment dans le cas des tumeurs solides résistantes.

La présente invention a plus particulièrement pour objet :

les composés de formule I:

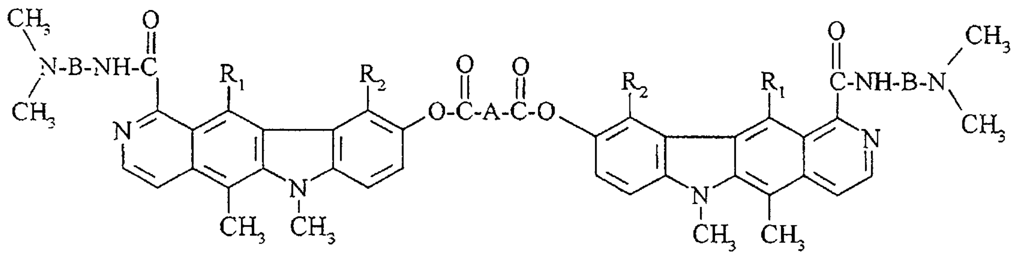

(I)

dans laquelle :

**$R_1$ et $R_2$,** identiques ou différents, représentent chacun un atome d'hydrogène ou un radical méthyle ;

**A** représente une chaîne hydrocarbonée, saturée, linéaire ou ramifiée contenant de 1 à 12 atomes de carbone pouvant éventuellement inclure un cycle hydrocarboné de 3 à 6 atomes de carbone ;

**A** peut également représenter un cycle hydrocarboné de 3 à 6 atomes de carbone ; et

**B** représente une chaîne hydrocarbonée, linéaire ou ramifiée, contenant de 1 à 8 atomes de carbone ;

ainsi que leurs éventuels isomères optiques, N oxydes et sels d'addition à un acide pharmaceutiquement acceptable.

L'invention a également pour objet le procédé de préparation des composés de formule I caractérisé en ce que l'on estérifie un composé de formule II :

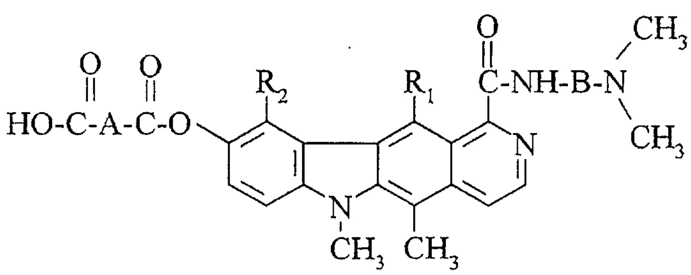

(II)

dans laquelle :

**$R_1$, $R_2$, A** et **B** ont les significations précédemment définies,

avec un composé de formule III :

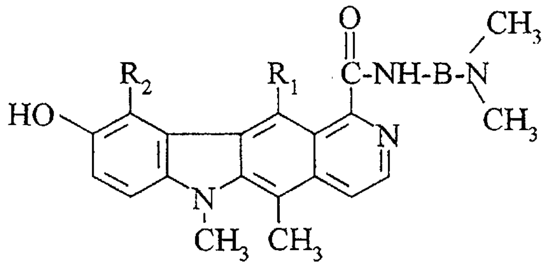

(III)

dans laquelle :

**$R_1$**, **$R_2$** et **B** ont les significations précédemment définies.

Il est particulièrement avantageux de faire réagir les composés de formule II avec les composés de formule III en présence d'un agent de couplage tel que l'hexafluorophosphate de benzotriazol-1-yloxy tris pyrrolidino phosphonium et d'une amine tertiaire telle que la triéthylamine dans un solvant aprotique polaire tel que le tétrahydrofurane, le diméthyl formamide ou la N-méthylpyrrolidone à une température comprise entre 0 et 80 °C.

Les composés II utilisés comme matières premières sont décrits dans la demande de brevet FR publiée sous le numéro 2.757.859.

Les composés III utilisés comme matières premières sont décrits dans la demande de brevet EP 0 591 058 A1 ou dans la demande de brevet FR publiée sous le numéro 2.757.858.

Les dérivés de formule I donnent des sels avec des acides physiologiquement tolérables, sels qui sont à ce titre inclus dans la présente invention.

Certains dérivés de formule I renferment un ou plusieurs carbones asymétriques et de ce fait, donnent des énantiomères ou des diastéréoisomères qui font également partie de la présente invention.

Les dérivés de la présente invention présentent des propriétés pharmacologiques particulièrement intéressantes, notamment une excellente cytotoxicité in vitro et une activité antitumorale in vivo supérieure à celle des produits de l'état antérieur de la technique, ce qui, permet leur utilisation en thérapeutique en tant qu'agents antitumoraux, particulièrement pour traiter les tumeurs solides résistantes.

La présente invention a également pour objet les compositions pharmaceutiques contenant comme principe actif au moins un composé de la présente invention mélangé ou associé à un ou plusieurs excipients pharmaceutiques ou véhicules inertes non toxiques.

Ces compositions pharmaceutiques sont généralement présentées sous forme dosée convenant pour l'administration par voie orale, rectale ou parentérale et notamment les comprimés, dragées, gélules, suppositoires et solutions injectables ou buvables.

La posologie varie selon l'âge et le poids du patient, la voie d'administration, la nature de l'indication thérapeutique et les traitements éventuellement associés et s'échelonne entre 0,1 et 400 mg par jour en une ou plusieurs administrations.

Les exemples suivants illustrent la présente invention, les points de fusion étant déterminés au tube capillaire. La silice utilisée pour les purifications par chromatographie sur colonne est la silice Amicon (0,035-0,07 mm). La pression utilisée est $10^5$ Pa.

## Exemple 1

Le bis-[1-(2-diméthylaminoéthylcarbamoyl)-5,6,11-triméthyl-6H-pyrido [4,3-b] carbazol -9-yl] ester de l'acide hexanedioïque.

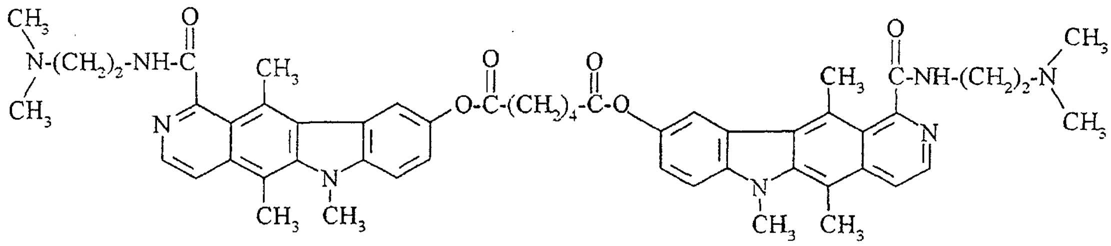

On dissous 2,6 g de [1-(2-diméthylaminoéthylcarbamoyl)-5,6,11-triméthyl-6H-pyrido [4,3-b] carbazol-9-yl] monoester de l'acide hexanedioïque, 2 g d'acide 9-hydroxy-5,6,11-triméthyl-6H-pyrido [4,3-b] carbazole-1-carboxylique (2-diméthylaminoéthyl)amide, 1,23 g de triéthylamine et 3,19 g d'hexafluorophosphate de benzotriazol-1-yloxy tris pyrrolidinophosphonium dans 40 ml de N-méthylpyrrolidone. On agite pendant 24 heures à température ambiante. On concentre à sec. On reprend le résidu dans un mélange de dichlorométhane, de solution aqueuse de carbonate de sodium et d'une quantité minimum de méthanol pour dissoudre l'insoluble. On décante, sèche la phase organique sur sulfate de sodium et concentre à sec. On chromatographie le résidu sur 280 g de silice en éluant avec un mélange de dichlorométhane, de méthanol et de triéthylamine (95-5-0,5). On concentre à sec les fractions contenant le produit et on lave le résidu à l'éthanol. On sèche à 40 °C sous vide et obtient 1,6 g de produit souhaité. PF 220-225 °C.

<u>**Exemples 2 à 10**</u>

En opérant comme décrit dans l'exemple 1, ont été préparés les composés objets des exemples suivants :

2) Le bis [1-(2-diméthylaminoéthylcarbamoyl)-5,6-diméthyl-6H-pyrido [4,3-b] carbazol-9-yl] ester de l'acide hexanedioïque. PF : 250-254 °C.

3) Le bis [1-(2-diméthylaminoéthylcarbamoyl)-5,6-diméthyl-6H-pyrido [4,3-b] carbazol-9-yl] ester de l'acide octanedioïque (gomme épaisse).

4) Le bis [1-(2-diméthylaminoéthylcarbamoyl)-5,6,10-triméthyl-6H-pyrido [4,3-b] carbazol-9-yl] ester de l'acide hexanedioïque. PF : 260-265 °C.

5) Le bis [1-(2-diméthylaminoéthylcarbamoyl)-5,6-diméthyl-6H-pyrido [4,3-b] carbazol-9-yl] ester de l'acide 3,3-diméthyl pentane dioïque.PF (dec) > 130 °C.

6) Le 1-(2-diméthylaminoéthylcarbamoyl)-5,6-diméthyl-6H-pyrido [4,3-b] carbazol-9-yl ester de l'acide {1-[1-(2-diméthylaminoéthylcarbamoyl)-5,6-diméthyl-6H-pyrido [4,3-b] carbazol-9-yloxycarbonylméthyl] cyclohexyl} acétique. PF : 145-150 °C.

7) Le bis [1-(2-diméthylaminoéthylcarbamoyl)-5,6-diméthyl-6H-pyrido [4,3-b] carbazol-9-yl] ester de l'acide 2,2,5,5-tétraméthyl hexanedioïque. PF : 112-120 °C.

8) Le bis [1-(2-diméthylaminoéthylcarbamoyl)-5,6-diméthyl-6H-pyrido [4,3-b] carbazol-9-yl] ester de l'acide cyclohexane-1,4-dicarboxylique. PF (dec) > 200 °C.

9) Le bis [1-(2-diméthylaminoéthylcarbamoyl)-5,6-diméthyl-6H-pyrido [4,3-b] carbazol-9-yl] ester de l'acide 3-méthyl pentanedioïque. PF : 115-120 °C.

10) Le bis [1-(3-diméthylaminopropylcarbamoyl)-5,6-diméthyl-6H-pyrido [4,3-b] carbazol-9-yl] ester de l'acide pentanedioïque et son trichlorhydrate qui est un produit amorphe.

**Exemple 11 : Etude pharmacologique**

**A/ Etude de cytotoxicité**

Trois lignées cellulaires ont été utilisées :

- 1 leucémie murine L 1210,
- 1 mélanome murin, B 16
- 1 carcinome pulmonaire humain, A 549.

Les cellules sont cultivées dans du milieu de culture RPMI 1640 complet contenant 10 % de sérum de veau foetal, 2 mM de glutamine, 50 unités/ml de pénicilline, 50 µg/ml de streptomycine et 10 mM d'Hepes, pH = 7,4.
Les cellules sont réparties dans des microplaques et exposées aux composés cytotoxiques. Les cellules sont ensuite incubées pendant 2 jours (L 1210) ou 4 jours (A 549, B 16). Le nombre de cellules viables est ensuite quantifié par un essai colorimétrique, le Microculture Tetrazolium Assay (Carmichael J., DeGraff W.G., Gazdar A.F., Minna J. D. and Mitchell J.R., Evaluation of a tetrazolium-based semiautomated colorimetric assay : assessment of chemosensitivity testing, Cancer Res., 47, 936-942, 1987).
Les résultats sont exprimés en $IC_{50}$, concentration en cytotoxique qui inhibe à 50 % la prolifération des cellules traitées. Les résultats obtenus sur les lignées utilisées sont rassemblés dans le tableau ci-après.
A titre d'exemple sont répertoriés ci-après les résultats obtenus avec le composé de l'exemple 1 particulièrement représentatif de l'invention et deux produits de référence :
le produit de référence A étant le composé objet de l'exemple 1 dans la demande de brevet EP 0 591 058 A1, soit le 1-(N,N-diméthylaminoéthylaminocarbamoyl)-5,6-diméthyl-9-hydroxy-6H-pyrido[4,3-b]carbazole, et le produit de référence **B** étant l'Adriamycine (ADR).

| *Cytotoxicité* | | | |
|---|---|---|---|
| ***Composés testés*** | ***$IC_{50}$ (nM)*** | | |
| | ***L 1210*** | ***B 16*** | ***A 549*** |
| ***Exemple 1*** | 73,5 | 3,0 | 17,6 |
| ***Référence A*** | 7,8 | 5,1 | 30,5 |
| ***Référence B*** | 24,3 | 6,8 | 39,4 |

La cytotoxicité du produit de l'exemple 1 de la présente invention est plus élevé sur les lignées B 16 et A 549 que sur la lignée L 1210, ce qui indique une meilleure activité in vitro pour les tumeurs solides. Sur ces deux lignées (B 16 et A 549), le produit de l'exemple 1 de la présente invention est plus cytotoxique que les produits de référence A et B.

**B/ Activité in vivo : Activité antitumorale**

La lignée P 388 (leucémie murine) a été fournie par le National Cancer Institute (Frederick, USA). Les cellules tumorales ($10^6$ cellules) ont été inoculées au jour 0 dans la cavité péritonéale de souris BDF1 femelles (Iffa-Credo, France) pesant de 18 à 20 g (groupes de 6 ou 7 animaux).
Les produits ont été administrés au jour 1 ou aux jours 1, 5, 9 aux doses indiquées, par voie intraveineuse.
L'activité antitumorale est exprimée en % de T/C :

$$\% \ T/C = \frac{\text{Temps de survie médian des animaux traités}}{\text{Temps de survie médian des animaux contrôles}} \times 100$$

Les animaux survivants à 60 jours (survivants à long terme) sont indiqués.
La lignée B 16 (mélanome murin) a été fournie par le National Cancer Institute (Frederick, USA). Cette tumeur est maintenue par greffes successives de fragments tumoraux en sous cutané. Au jour 0, les tumeurs sont broyées et homogénéisées dans du NaCl à 0,9 % (1 g de tumeur dans 10 ml) et 0,5 ml de l'homogénat sont injectés dans la cavité péritonéale de chaque souris BDF1. Les produits ont été administrés aux doses indiquées, 1 fois par jour pendant 9 jours ($J_{1-9}$) par voie intra-péritonéale.
L'activité antitumorale est exprimée en % de T/C, comme défini ci-dessus. Les animaux survivants à 90 jours (survivants

à long terme) sont indiqués.

Résultats

Le produit de l'exemple 1 de la présente invention est plus puissant et aussi actif que le produit de référence A sur le modèle de la leucémie P 388.

Sur le modèle du mélanome B 16, le produit de l'exemple 1 de la présente demande est très actif, il induit 3 survivants à long terme à la dose optimale, alors que le produit de référence A n'en induit aucun. Le produit de l'exemple 1 de la présente demande est donc très intéressant contre les tumeurs solides résistantes.

| ***Activité antitumorale in vivo*** | | | | | | |
|---|---|---|---|---|---|---|
| ***Tumeur*** | ***Produit*** | ***Schéma*** | ***Voie*** | ***Dose optimale*** | ***T /C, %*** | ***Survivants à long terme / nb total de souris*** |
| P 388 i.p | Exemple 1 | $J_{1}$ | i.v. | 10 mg/kg | 252 | 1/6 |
| | | $J_{1,5,9}$ | i.v. | 10 mg/kg | > 582 | 3/6 |
| | Référence A | $J_{1}$ | i.v. | 80 mg/kg | 221 | 0/6 |
| | | $J_{1,5,9}$ | i.v. | 80 mg/kg | 320 | 2/6 |
| B 16 i.p. | Exemple 1 | $J_{1-9}$ | i.p. | 5 mg/kg | 429 | 3/7 |
| | Référence A | $J_{1-9}$ | i.p. | 10 mg/kg | 190 | 0/7 |

## Revendications

**1.** Les composés de formule I :

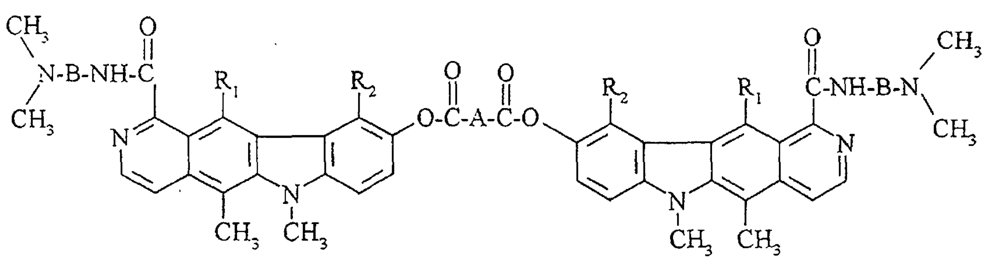

(I)

dans laquelle :

**$R_1$ et $R_2$,** identiques ou différents, représentent chacun un atome d'hydrogène ou un radical méthyle ;

**A** représente une chaîne hydrocarbonée, saturée, linéaire ou ramifiée contenant de 1 à 12 atomes de carbone pouvant éventuellement inclure un cycle hydrocarboné de 3 à 6 atomes de carbone ;

**A** représente également un cycle hydrocarboné de 3 à 6 atomes de carbone ; et

**B** représente une chaîne hydrocarbonée, linéaire ou ramifiée, contenant de 1 à 8 atomes de carbone ;

ainsi que leurs isomères optiques, N oxydes et sels d'addition à un acide pharmaceutiquement acceptable.

**2.** Un composé de la revendication 1 qui est le bis-[1-(2-diméthylaminoéthylcarbamoyl)-5,6,11-triméthyl-6H-pyrido [4,3-b] carbazol -9-yl] ester de l'acide hexanedioïque.

**3.** Le procédé de préparation des composés de la revendication 1 **caractérisé en ce que** l'on estérifie un composé de formule II :

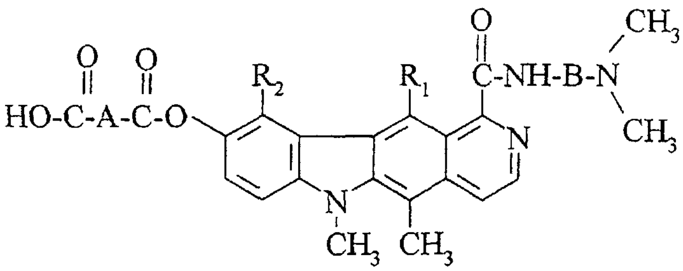

(II)

dans laquelle :

$R_1$, $R_2$, **A** et **B** ont les significations définies dans revendication 1, avec un composé de formule III :

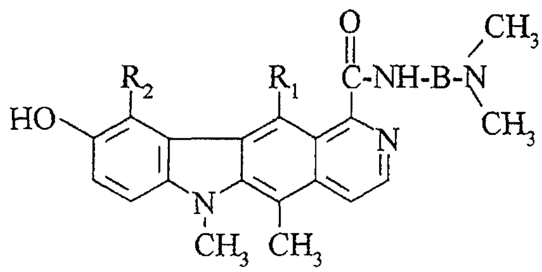

(III)

dans laquelle :
$R_1$, $R_2$ et **B** ont les significations définies dans la revendication 1.

**4.** Les compositions pharmaceutiques, utiles comme agents antitumoraux, contenant comme principe actif au moins un composé selon l'une quelconque des revendications 1 et 2, mélangé ou associé à un ou plusieurs excipients pharmaceutiques ou véhicules inertes non toxiques.

**Patentansprüche**

**1.** Verbindungen der Formel I:

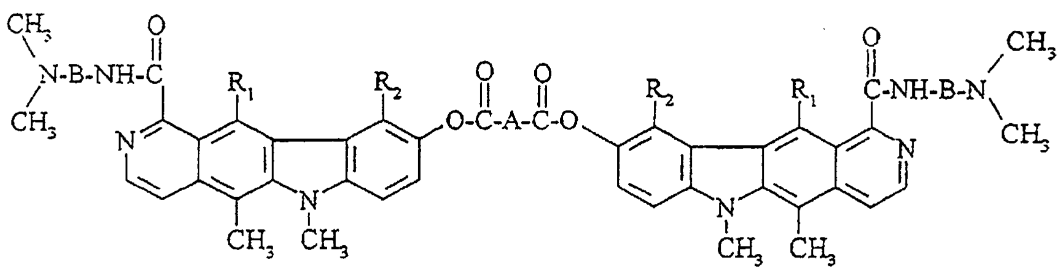

(I)

in der:

| | |
|---|---|
| $R_1$ und $R_2$, | die gleichartig oder verschieden sind, jeweils ein Wasserstoffatom oder eine Methylgruppe bedeuten; |
| **A** | eine gesättigte, geradkettige oder verzweigte Kohlenwasserstoffkette, die 1 bis 12 Kohlenstoffatome enthält und gegebenenfalls einen Kohlenwasserstoffring mit 3 bis 6 Kohlenstoffatomen einschließen kann, bedeutet; |

**A** ebenfalls einen Kohlenwasserstoffring mit 3 bis 6 Kohlenstoffatomen darstellt; und
**B** eine geradkettige oder verzweigte Kohlenwasserstoffkette, die 1 bis 8 Kohlenstoffatome enthält, bedeutet,

sowie deren optische Isomere, deren N-Oxide und deren Additionssalze mit einer pharmazeutisch annehmbaren Säure.

2. Verbindung nach Anspruch 1, nämlich Bis[1-(2-dimethylaminoethylcarbamoyl)-5,6,11-trimethyl-6H-pyrido[4,3-b]carbazol-9-yl]-ester der Hexandisäure.

3. Verfahren zur Herstellung der Verbindungen nach Anspruch 1, **dadurch gekennzeichnet, daß** man eine Verbindung der Formel II:

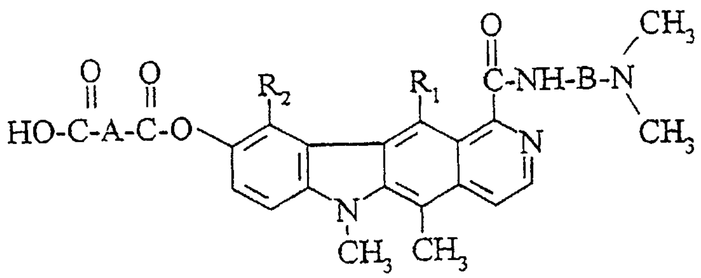

(II)

in der:

**$R_1$, $R_2$, A** und **B** die in Anspruch 1 angegebenen Bedeutungen besitzen,
mit einer Verbindung der Formel III:

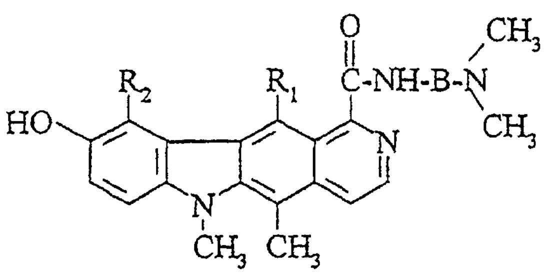

(III)

in der:
**$R_1$, $R_2$** und **B** die in Anspruch 1 angegebenen Bedeutungen besitzen, verestert.

4. Pharmazeutische Zubereitungen, die als antitumorale Mittel nützlich sind, enthaltend als Wirkstoff mindestens eine Verbindung nach einem der Ansprüche 1 und 2 in Mischung oder in Kombination mit einem oder mehreren inerten, nichttoxischen pharmazeutischen Trägermaterialien oder Bindemitteln.

## Claims

1. Compounds of formula I:

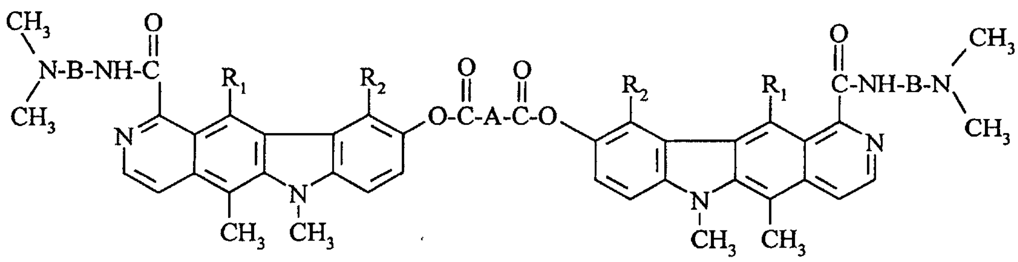

(I)

in which:

**$R_1$ and $R_2$,** which may be the same or different, each represents a hydrogen atom or a methylradical;

**A** represents a linear or branched, saturated hydrocarbon chain containing from 1 to 12 carbon atoms optionally including a hydrocarbon ring having from 3 to 6 carbon atoms;

**A** also represents a hydrocarbon ring having from 3 to 6 carbon atoms; and

**B** represents a linear or branched hydrocarbon chain containing from 1 to 8 carbon atoms;

as well as their possible optical isomers, N-oxides and addition salts with a pharmaceutically acceptable acid.

2. A compound of claim 1 which is the bis[1-(2-dimethylaminoethylcarbamoyl)-5,6,11-trimethyl-6H-pyrido[4,3-b]carbazol-9-yl] ester of hexanedioic acid.

3. Process for the preparation of compounds of claim 1, **characterised in that** a compound of formula II :

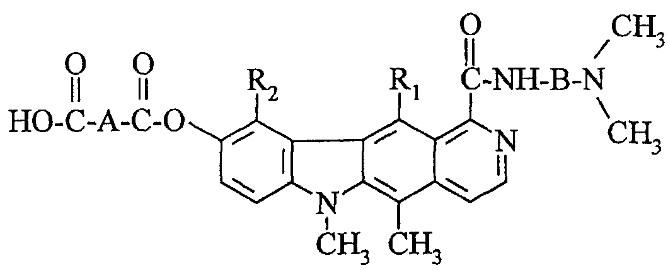

(II)

in which:

**$R_1$, $R_2$, A** and **B** are as defined in claim 1,
is esterified by a compound of formula III :

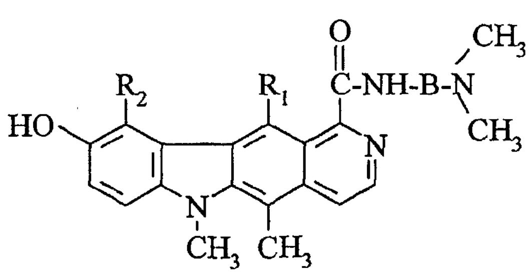

(III)

in which:
**$R_1$, $R_2$** and **B** are as defined in claim 1.

**4.** Pharmaceutical compositions, for use as anti-tumour agents, comprising as active ingredient at least one compound according to any one of claims 1 and 2, in admixture or in association with one or more pharmaceutical excipients or inert, non-toxic carriers.